(19) Europäisches Patentamt
European Patent Office
Office européen des brevets

(11) Publication number: **0 101 944**
**B1**

(12) **EUROPEAN PATENT SPECIFICATION**

(45) Date of publication of patent specification: **18.09.85**

(51) Int. Cl.⁴: **C 07 C 59/48,** A 61 K 31/19

(21) Application number: **83107428.1**

(22) Date of filing: **28.07.83**

(54) **Immunomodulators containing as the active ingredient alpha-benzyl malic acid.**

(30) Priority: **30.07.82 JP 134409/82**

(43) Date of publication of application:
**07.03.84 Bulletin 84/10**

(45) Publication of the grant of the patent:
**18.09.85 Bulletin 85/38**

(84) Designated Contracting States:
**AT BE CH DE FR GB IT LI NL SE**

(56) References cited:

**CHEMICAL ABSTRACTS, vol. 80, no. 17, 29th April 1974, p. 436, no. 96192q, Columbus, Ohio (USA); S.BRANDANGE et al.: "Orchidaceae alkaloids. XXXVIII. Asymmetric synthesis of 2-isobutylmalic acid and 2-(cyclohexylmethyl)malic acid"**

**CHEMICAL ABSTRACTS, vol. 80, no. 25, 24th June 1974, p. 381, no. 14534x, Columbus, Ohio (USA); S.BRANDANGE et al.: "Absolute configurations of 2-alkylmalic acids"**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotama-kita Nerima-ku**
**Tokyo (JP)**
Inventor: **Takeuchi, Tomio**
**5-1-11, Higashi-Gotanda Shinagawa-ku**
**Tokyo (JP)**
Inventor: **Aoyagi, Takaaki**
**3-3-6, Honkugenuma**
**Fujisawa-City Kanagawa Prefecture (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

(56) References cited:
**CHEMICAL ABSTRACTS, vol. 82, no. 13, 31st March 1975, p. 263, no. 83073t, Columbus, Ohio (USA); D.DOERNEMANN et al.: "Chain elongation of aromatic acids. Role of 2-benzylmalic acid in the biosynthesis of a C6C4 amino acid and a C6C3 mustard oil glucoside"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 101 944 B1

**Description**

This invention relates to novel immunomodulators, and more specially, to immunomodulators containing as the active ingredient α-benzyl malic acid (hereinafter referred to as the present compound) represented by the following formula:

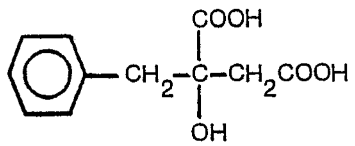

After extensive study of the pharmacological actions of various compounds, particularly, their actions on cell-mediated immunity, it has now been found that the present compound has a marked effect of potentiating cell-mediated immunity.

The present compound, α-benzyl malic acid, is a known compound in the literature, and its physical and chemical properties have been known. However, the pharmacological action of the present compound, particularly, its effect of enhancing cell-mediated immunity has been found out for the first time after elaborated investigation.

The present compound can be synthesized by various methods. Their examples include the method described in Bioorganic Chemistry 5, 1977—186 (1976).

This method relates to the synthesis of the present compound in accordance with the following reaction scheme using 3-carboxy-3-butenoate (I) as a starting compound:

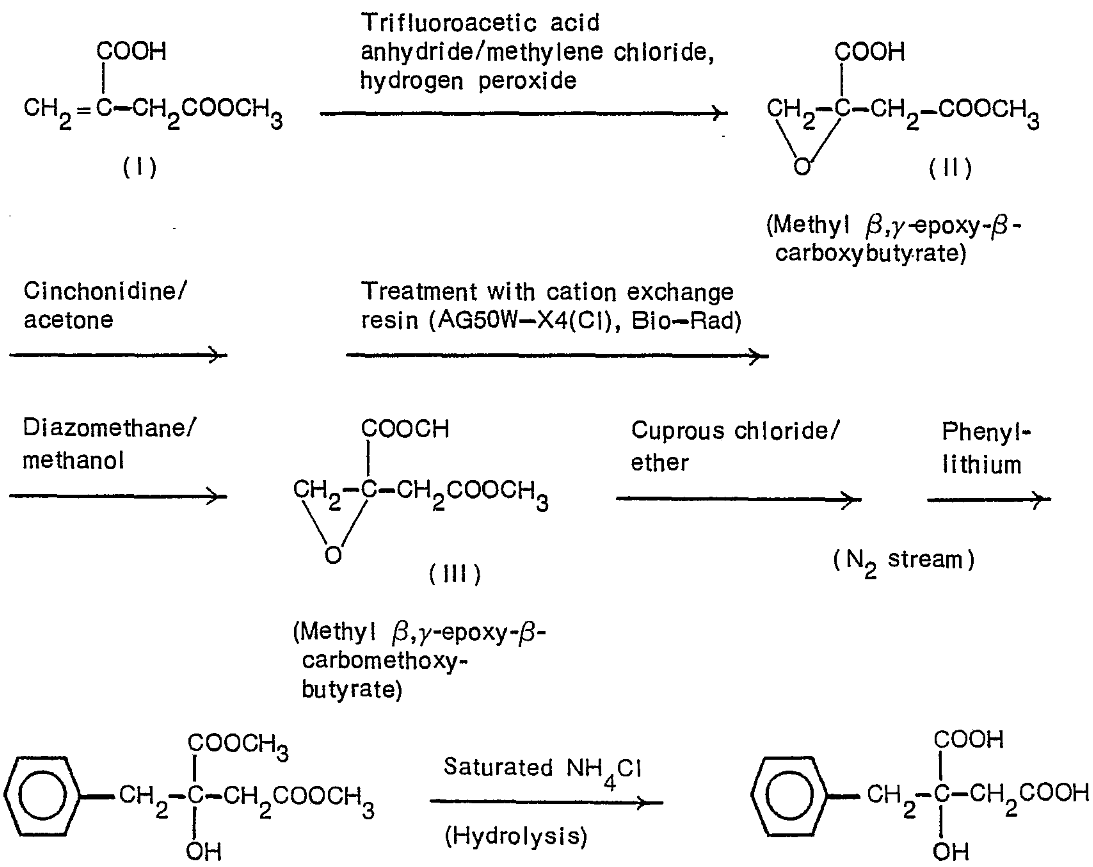


(I)

(II)

(Methyl β,γ-epoxy-β-carboxybutyrate)

(III)

(Methyl β,γ-epoxy-β-carbomethoxy-butyrate)

(IV)

(Dimethyl α-benzyl malate)

(V)

(α-benzyl malic acid)

The biological activity of the present compound will be explained by reference to the following experimental examples.

Experimental Example 1
(Effect on cell-mediated immunity in normal mice)

(i) Method:

The action of α-benzyl malic acid on cell-mediated immunity was investigated using as its index the degree of delayed-type hypersensitivity (hereinafter referred to as D.T.H.) obtained by inoculating the foot pads of mice with sheep red blood cells (hereinafter referred to as SRBC) used as the antigen (Reference: J.Exp.Med. 139, 1529—1539, 1974).

A supension of $10^8$ SRBCs in 0.05 ml of physiological saline solution was inoculated subcutaneously to the foot pad of one of the hind legs of $CDF_1$ mice (female, 8 weeks old). Simultaneously, an aqueous solution containing 5, 0.5, 0.05 or 0.005 mg/kg of α-benzyl malic acid was administered intraperitoneally in a single dose. Four days after administration, a suspension of $10^8$ SRBCs in 0.05 ml of physiological saline solution was subcutaneously administered to the foot pad of the other hind leg to induce secondary immune response. Twenty-four hours later, swelling on the foot pad (increased thickness of the foot pad) was measured with a calipers. The thickness of the foot pad in control animals, which had been sensitized with SRBCs and intraperitoneally administered physiological saline solution without having received the test compound, was evaluated to be 100%. The thickness of the foot pad in the test animals that had been treated in the aforementioned way was compared with that in the control animals, thereby judging the cell-mediated immunity potentiating effect of the test compound.

(ii) Results:

| Test compounds | Dose (mg/kg) | Thickness of the foot pad (× 0.1 mm) | % on the control's value |
|---|---|---|---|
| α-benzyl malic acid | 5 | 12.8±1.42 | 149 |
| | 0.5 | 14.4±1.52 | 167 |
| | 0.05 | 13.4±1.61 | 156 |
| | 0.005 | 11.8±1.15 | 137 |
| *Bestatin | 0.5 | 12.6±1.12 | 147 |
| Control | | 8.6±1.29 | 100 |

*Bestatin: [(2S,3R)-5-amino-2-hydroxy-4-phenyl-(S)-leucine], a compound having immunomodulating activity which was made public by Japanese Patent Application Laid-Open No. 7187/76.

As noted above, the present compound was found to have a cell-mediated immunity augmenting activity comparable or superior to that (147%) for the group receiving 0.5 mg/kg of the reference drug bestatin.

Experimental Example 2
(Effect on cell-mediated immunity in cancer-inflicted mice; picryl chloride was used as the antigen)

(i) Method:

$10^6$ cells of Ascites Sarcoma 180 were intraperitoneally transplanted to groups of 12-week-old female $CDF_1$ mice (6 mice/group). The day of transplantation was designated as Day 0. On Day 1, a 6% solution of picryl chloride in ethanol was applied to a shaven skin (25 mm × 15 mm) of the abdomen with the use of a cut absorbent cotton (20 mm × 20 mm × 2 mm) impregnated with the solution to thereby sensitize the mice. On Day 8, both ears were measured for thickness with a dial gauge, to determine the baseline value (a).

Then, both ears were sensitized with a 20 mm × 4 mm × 1 mm cut absorbent cotton impregnated with a 1% solution of picryl chloride in olive oil. On Day 9, both swollen ears were measured for their thickness with a dial gauge to obtain a value (b). The baseline value (a) was subtracted from the value (b) to determine the increase in ear thickness (b−a) for the control group (C).

On the other hand, 5.0, 0.5, 0.05 or 0.005 mg/kg of the test compound dissolved in physiological saline solution was orally administered once daily for 6 consecutive days between the day and 8 days after inoculation of Sarcoma 180. The mice were sensitized with picryl chloride in the same way as in the control group, and the thickness of the ears was similarly measured to determine the increase in ear thickness (b'−a') for the treatment group (T).

The rate of increase in ear thickness for the treatment group to that for the control group was calculated from the following equation:

$$\frac{T}{C} \times 100$$

to determine the cell-mediates immunmity modulating activity of the present compound.

( ii ) Results:

| Test compounds | Dose (mg/kg) | Increase in ear thickness (x 0.01 mm) | Rate to the control value (%) |
|---|---|---|---|
| α-benzyl malic acid | 5.0 | 4.71 ± 0.95 | 114.6 |
| | 0.5 | 5.08 ± 0.56 | 123.6 |
| | 0.05 | 5.29 ± 0.33 | 128.7 |
| | 0.005 | 5.18 + 0.67 | 126.0 |
| Control (physiological saline) | | 4.11 ± 1.11 | 100.0 |

$P < 0.05$

Thus, the group treated with 0.05 mg/kg of the present compound showed significant cell-mediated immunity modulating activity corresponding to an increase in ear thickness of 5.29 ± 0.33 ($10^{-2}$ mm) and a rate of increase in ear thickness of 128.7%.

The foregoing results demonstrate that α-benzyl malic acid potentiates cell-mediated immunity in normal animals and modulates cell-mediated immunity depressed by cancer infliction.

Acute toxicity tests in mice have proved the present compound to cause no deaths at an i.v. dose of 500 mg/kg. The present compound is therefore believed to be a safe substance.

As noted above, α-benzyl malic acid of this invention has been found to enhance immune response by itself and to exhibit an anticancer effect mediated by the host. Accordingly, the compounds of this invention are useful as immunopotentiators and antitumor immunomodulators, or as adjuvants to various chemotherapeutic agents for the treatment of cancer.

Such drugs containing α-benzyl malic acid as their active ingredients can be prepared by blending either α-benzyl malic acid or its pharmacologically acceptable salts with widely used carriers, and if desired, further mixing the resulting blends with various chemotherapeutic agents. Examples of the α-benzyl malic acid salts are salts formed between the carboxyl groups of α-benzyl malic acid and pharmacologically acceptable cations, e.g., the cations of alkali metals, such as sodium and potassium, the cations of alkaline earth metals such as calcium and magensium, and ammonium ions (i.e. carboxylates).

The present compound or drugs of this invention may be administered as oral preparations, injections or rectal suppositories. Compositions comprising the aforementioned active ingredient can be converted into lyophilized injections by additing to them pH adjustors, buffers, stabilizers or excipients, and freeze-drying the mixtures by customary methods.

Hypodermic, intramuscular, and intravenous injections can be prepared from compounds of the active ingredient by customary methods after the addition of pH adjustors, buffers, stabilizers, isotonizers, and local anesthetics. For the preparation of oral solids, excipients, and if desired, binders, disintegrators, lubricants, colorants, correctives of taste and correctives of odor may be added to compounds of the active ingredient, and the mixtures can be formed into tablets, coated tablets, granules, powders, capsules, etc. by customary methods.

For the preparation of oral liquids, correctives of taste, buffers, stabilizers, correctives of odor, etc. may be added to compounds of the active ingredient, and the mixtures can be formed into syrups and dry syrups by customary methods. To prepare rectal suppositories, excipients, and if desired, surfactants may be added to compounds of the active ingredient, and the mixtures can be made into suppositories by customary methods.

The dose of α-benzyl malic acid may be varied depending on symptoms. For adults, it is recommended to administer 0.02 to 200 mg of α-benzyl malic acid once daily. For concomitant therapy with other carcinostatic chemotherapeutic agents or immunostimulants, α-benzyl malic acid in doses within said dosage range can be combined with the usual doses of such carcinostatic chemotherapeutic agents or immunostimulants.

Examples of preparations, according to the present invention, are shown hereinbelow:

Example 1

1 g of sodium α-benzyl malate and 5 g of mannitol are dissolved in distilled water to make 1,000 ml of a solution. The solution is sterilized by a customary method, and dispensed into vials into amonts of 2 ml

each, followed by freeze-drying the contents. When the lyophilized product is to be used, it is reconstituted with distilled water to make an injection.

Example 2

1 part of sodium α-benzyl malate, 200 parts of lactose, 50 parts of corn starch, and 7 parts of polyvinyl pyrrolidone are mixed. The mixture is granulated with ethanol, dried, and screened by customary methods. The resulting granules are blended with 0.5% magnesium stearate, and the blend is formed by a tablet press into tablets with an active ingredient content of 3.6 mg each.

Example 3

0.5 g of sodium α-benzyl malate, 300 g of sugar, 1 g of citric acid, 250 mg of thioglycerol and 2 ml of orange extracts are dissolved in distilled water to make 1,000 ml of a solution which serves as a syrup.

Example 4

1 part of sodium α-benzyl malate and 300 parts of cacao butter are fused and blended. The blend is formed by a customary method into suppositories weighing 2 g each.

**Claims for the Contracting States: BE CH DE FR GB IT LI LU NL SE**

1. A pharmaceutical composition comprising as active ingredient α-benzyl malic acid of the formula

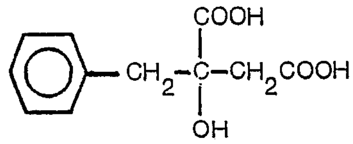

and/or a pharmacologically acceptable salt thereof in association with a pharmaceutically acceptable carrier and/or auxiliary.

2. α-Benzyl malic acid of the formula

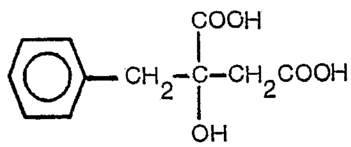

for use in potentiating cell-mediated immunity.

3. α-Benzyl malic acid of the formula as defined in claims 1 and 2 for use as antitumor immunomodulator.

4. α-Benzyl malic acid of the formula as defined in claims 1 and 2 for use as an adjuvant for the treatment of cancer.

**Claim for the Contracting State: AT**

A process for the preparation of pharmaceutical compositions effective for potentiating cell-mediated immunity or as antitumor immunomodulator, characterized in that α-benzyl malic acid of the formula

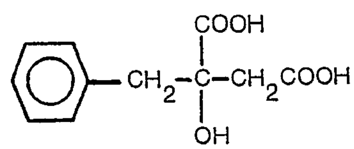

and/or a pharmacologically acceptable salt thereof is brought, together with at least one solid, liquid or semi-liquid carrier or auxiliary and, optionally with a further active ingredient into a suitable dosage form.

**Patentansprüche für die Vertragsstaaten: BE CH DE FR GB IT LI NL SE**

1. Eine pharmazeutische Zubereitung enthaltend als Wirkstoff α-Benzyläpfelsäure der Formel

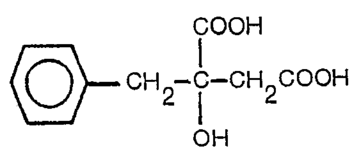

und/oder eines ihrer pharmakologisch verträglichen Salze in Verbindung mit einem pharmazeutish verträglichen Träger- und/oder Hilfsstoff.

2. α-Benzyläpfelsäure der der Formel

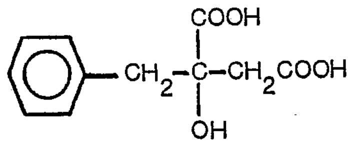

zur Verwendung bei der Potenzierung der zellkodierten Immunität.

3. α-Benzyläpfelsäure der in den Ansprüchen 1 und 2 definierten Formel zur Verwendung als ein Tumorwachstum hemmender Immunomodulator.

4. α-Benzyläpfelsäure der in den Ansprüchen 1 und 2 definierten Formel als Adjuvans bei der medikamentösen Behandlung von Krebserkrankungen.

**Patenansprüche für den Vertragsstaat: AT**

1. Ein Verfahren zur Herstellung von pharmazeutischen zubereitungen mit der Wirkung der Potenzierung von zellkodierter Immunität und Hemmung des Wachstums von Tumoren, dadurch gekennzeichnet, daß α-Benzyläpfelsäure der Formel

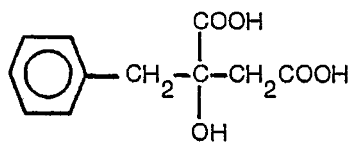

und/oder eines ihrer pharmakologisch verträglichen Salze zusammen mit wenigstens einem Feststoff, einer Flüssigkeit oder einem halb-festen Träger oder Hilfsstoff und gegebenenfalls mit einem weiteren Wirkstoff in eine geeignete Dosierungsform gebracht wird.

**Revendications pour les Etats contractants: BE CH DE FR GB IT LI LU NL SE**

1. Composition pharmaceutique comprenant, comme ingrédient actif, de l'acide α-benzyl malique de formule:

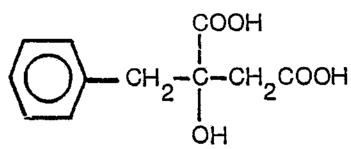

et/out un sel pharmacologiquement acceptable de cet acide en association avec un support et/ou un adjuvant pharmaceutiquement acceptable.

2. L'acide α-benzyl malique de formule:

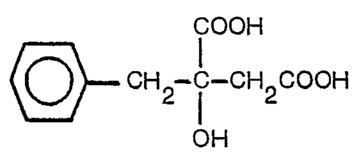

destiné à être utilisé pour potentialiser l'immunité par médiation cellulaire.

3. L'acide α-benzyl malique de formule telle que définie dans les revendications 1 et 2 destiné à être utilisé comme immunomodulateur antitumoral.

4. L'acide α-benzyl malique de formule telle que définie dans les revendications 1 et 2 destiné à être utilisé comme adjuvant dans le traitement du cancer.

**Revendications pour l'Etat contractant: AT**

1. Procédé de préparation de compositions pharmaceutiques efficaces pour potentialiser l'immunité par médiation cellulaire ou comme immunomodulateur anti-tumoral, caractérisé en ce que l'acide α-benzylmalique de formule:

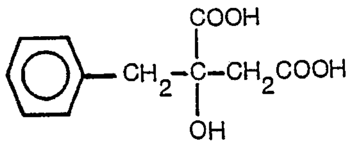

et/ou un sel pharmacologiquement acceptable de cet acide, sont amenés, avec au moins un support ou adjuvant solide, liquide ou semi-liquide et, éventuellement, avec un autre ingrédient actif dans une forme posologique appropriée.